# FASCICULE DE BREVET EUROPEEN

(11) **EP 0 701 429 B1**
(45) Date de publication et mention de la délivrance du brevet: **23.04.1997**
(21) Numéro de dépôt: 94917067.4
(22) Date de dépôt: 31.05.1994
(51) Int. Cl.: A61K 7/50, A61K 7/48

(54) **COMPOSITIONS COSMETIQUES CONTENANT AU MOINS UN TENSIOACTIF ANIONIQUE DU TYPE ALKYLGALACTOSIDE URONATE ET AU MOINS UN ORGANOPOLYSILOXANE**
KOSMETISCHE MITTEL ENTHALTENDE MINDESTENS EINEN ANIONISCHEN TENSIDE VOM TYP ALKYLGALAKTRIDE-URONAT UND MINDESTENS EINEN ORGANOPOLYSILOXANEN
COSMETIC COMPOUNDS CONTAINING AT LEAST ONE ANIONIC ALKYLGALACTOSIDE URONATETYPE SURFACE-ACTIVE AGENT AND AT LEAST ONE ORGANOPOLYSILOXANE

(30) Priorité: 01.06.1993 FR 9306528
(43) Date de publication de la demande: 20.03.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400629
(87) Numéro de publication internationale: WO9427571

(56) Documents cités:
- EP-A- 0 550 276
- FR-A- 2 679 563

## Description

L'invention concerne des compositions cosmétiques contenant au moins un tensio-actif anionique du type alkylgalactoside uronate et un organopolysiloxane ainsi que leurs utilisations pour le traitement des matières kératiniques.

Les compositions de lavage des cheveux ou de la peau sont généralement formulées à partir d'agents tensio-actifs anioniques ou non-ioniques ou leurs mélanges en présence éventuellement de tensio-actifs amphotères.

On a proposé de rajouter à ces compositions de lavage, des silicones afin d'améliorer leurs propriétés cosmétiques, mais le pouvoir moussant de telles compositions et la qualité des mousses sont insuffisants.

Les tensio-actifs anioniques du type alkylgalactoside uronate ont déjà été préconisés dans des compositions lavantes pour les cheveux. Ils ont été décrits dans la demande de brevet EP 0 532 370.

Les compositions de lavage des cheveux utilisant uniquement ces tensio-actifs ne conduisent pas à de bonnes propriétés cosmétiques en particulier le démêlage des cheveux mouillés est difficile et les qualités des mousses sont insuffisantes.

La demanderesse vient de découvrir de manière surprenante, que l'association, dans des compositions lavantes et/ou traitantes pour matières kératiniques, d'un tensio-actif anionique du type alkylgalactoside uronate et d'un organopolysiloxane défini ci-après, confèrait à ces compositions des propriétés de démêlage des cheveux mouillés considérablement améliorées.

Par ailleurs, l'association conforme à la présente invention permet d'obtenir une mousse abondante, compacte et très douce.

En outre, la demanderesse a constaté que les compositions cosmétiques contenant une telle association conféraient de bonnes propriétés cosmétiques telles que la douceur, un toucher agréable et qu'elles étaient très bien tolérées par la peau.

La présente invention a donc pour objet des compositions cosmétiques contenant au moins un alkylgalactoside uronate et un organopolysiloxane défini ci-après.

Un autre objet de l'invention est constitué par l'utilisation de ces compositions pour le traitement et/ou le lavage des matières kératiniques telles que les cheveux ou la peau.

Enfin, un autre objet de l'invention consiste en un procédé de traitement cosmétique des cheveux ou de la peau au moyen des compositions de l'invention; les procédés de lavage et de traitement des cheveux étant préférés.

Les compositions cosmétiques selon l'invention contiennent dans un milieu aqueux cosmétiquement acceptable :
(**A**) au moins un agent tensio-actif anionique du type alkylgalactoside uronate de formule : 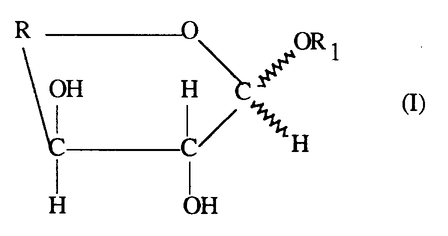 dans laquelle :
   R₁ désigne un radical alkyle linéaire ou ramifié de 8 à 22 atomes de carbone.
   R désigne un groupe
      (i) 〉CH -CH(OH) - CO₂R₂ ou
      (ii) 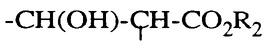 dont le carbone portant le groupe hydroxyle est relié à l'atome d'oxygène endocyclique; R₂ étant l'hydrogène, un métal alcalin, un métal alcalino-terreux ou un groupe ammonium quaternaire non substitué ou substitué par des radicaux alkyle, hydroxyalkyle ou dérivé d'aminoacides.
(**B**) au moins un organopolysiloxane choisi parmi :
   (i) - les huiles d'organopolysiloxanes à l'exclusion des polydiméthylsiloxanes linéaires de viscosité inférieure à 10⁻¹m²/s,
   (ii) - les gommes d'organopolysiloxanes,
   (iii) - les résines d'organopolysiloxanes
   (iv) les polysiloxanes organomodifiés
   (v) - les mélanges de polydiméthylsiloxanes linéaires de viscosité inférieure à 10⁻¹m²/s avec un ou plusieurs composé(s) défini(s) sous (i), (ii), (iii) et (iv).

Les tensio-actifs anioniques du type alkylgalactoside uronate de formule (I) sont connus et peuvent être préparés selon les procédés décrits dans la demande de brevet EP-A-0 532 370.

Le métal alcalin est notamment le sodium, le potassium et le métal alcalino-terreux est de préférence le magnésium. Comme sels d'ammonium quaternaires, on peut citer les sels d'ammoniaque, de triéthanolamine, de monoéthanolamine, de 2-amino 2-méthyl 1,3-propanediol, de 2-amino 2-méthyl 1-propanol; l'amino acide est notamment : l'histidine, l'arginine ou la lysine.

On utilise de préférence les composés de formule (I) pour lesquels le radical R₁ désigne un alkyle en C₈-C₁₄ et plus particulièrement le radical décyle.

On utilise notamment les composés suivants :
Décyl α-D-galactopyrioside uronate de sodium : 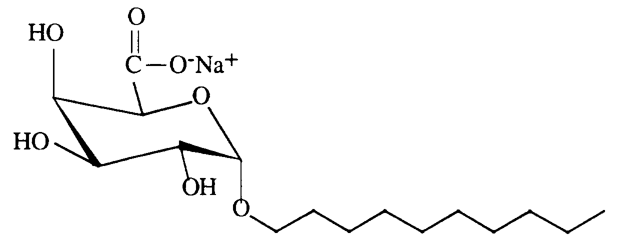
Décyl β-D-galactopyranoslde uronate de sodium : 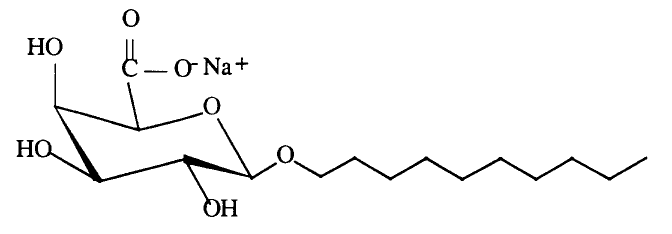
Décyl α-D-galactofuranoside uronate de sodium : 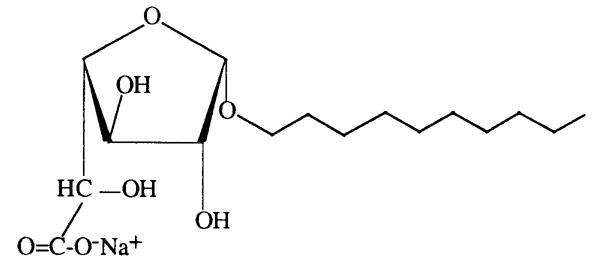
Décyl β-D-galactofuranoside uronate de sodium :

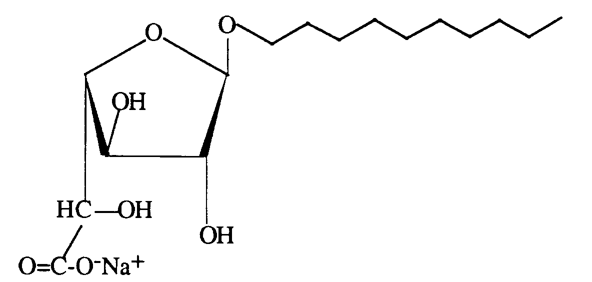

Les organopolysiloxanes utilisés dans les compositions selon la présente invention, sont des huiles d'organopolysiloxanes ou des gommes ou des résines d'organosiloxanes, telles quelles ou sous forme de solutions dans des solvants organiques ou encore sous forme d'émulsions ou de microémulsions.

Parmi les organosiloxanes utilisés conformément à la présente invention, on peut citer à titre non limitatif :

### I. Les silicones volatiles

Celles-ci possèdent un point d'ébullition compris entre 60°C et 260°C. Elles sont choisies parmi les silicones cycliques de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges.

On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane;

### II. Les silicones non volatiles

Elles sont constituées principalement par les polyalkylsiloxanes, les polyarylsiloxanes, les polyalkylarylsiloxanes, les gommes et résines de silicone et les polysiloxanes organomodifiés, ainsi que leurs mélanges.

Parmi les polyalkylsiloxanes, on peut citer principalement les polydiméthylsiloxanes linéaires de viscosité supérieure à 10⁻¹m²/s : soit,
- à groupements terminaux triméthylsilyle, comme par exemple, et à titre non limitatif, les huiles SILBIONE de la série 70047 commercialisées par RHONE POULENC; l'huile 47 V 500 000 de RHONE POULENC ou certaines Viscasil de la GENERAL ELECTRIC, ou
- à groupements terminaux hydroxydiméthylsilyle, telles que les huiles de la série 48 V de RHONE POULENC.

Dans cette classe de polyalkylsiloxanes, on peut également mentionner les polyalkylsiloxanes vendus par la Société GOLDSCHMIDT sous les dénominations "ABILWAX 9800" et "ABILWAX 9801", qui sont des polyalkyl (C₁-C₂₀) siloxanes.

Parmi les polyalkylarylsiloxanes, on peut citer les polyméthylphénylsiloxanes, les polydiméthylméthylphénylsiloxanes, les polydiméthyldiphénylsiloxanes linéaires et/ou ramifiés, tels que par exemple :
- l'huile RHODORSIL 763 de RHONE POULENC,
- les huiles SILBIONE 70641 V 30 et 70641 V 200 de RHONE POULENC,
- le produit DC 556 Cosmetic Grad Fluid de DOW CORNING,
- les silicones des séries PK de BAYER, telles que la PK20,
- les silicones des séries PN, PH de BAYER, comme les PN 1000 et PH 1000,
- certaines huiles des séries SF de GENERAL ELECTRIC, telles que les SF 1250, SF 1265, SF 1154, SF 1023.

Les gommes de silicone, conformes à la présente invention, sont des polydiorganosiloxanes de forte masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane))(phénylméthylsiloxane)],
- poly[(diméthylsiloxane)/(diphénylsiloxane)/(méthylvinylsiloxane)].

On peut citer, par exemple, à titre non limitatif, les mélanges suivants :
**1**) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit Q2 1401 vendu par la Société DOW CORNING :
**2**) les mélanges formés à partir d'une gomme polydiméthylsiloxane avec une silicone cyclique, tel que le produit SF 1214 SILICONE FLUID de GENERAL ELECTRIC, qui est une gomme SE 30 de PM 500 000 solubilisée dans la SF 1202 SILICONE FLUID (décaméthylcyclopentasiloxane);
**3**) les mélanges de deux PDMS de viscosité différente, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 m²/s et d'une huile SF 96 d'une viscosité de 5.10⁻⁶ m²/s (15% de gomme SE 30 et 85% d'huile SF 96).

Le produit CF 1241 est le mélange d'une gomme SE 30 (33%) et d'une PDMS (67%) de viscosité 10⁻³m²/s.

Les résines d'organopolysiloxanes utilisables conformément à l'invention, sont des systèmes siloxaniques réticulés renfermant les unités :
R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593" ou ceux vendus sous les dénominations "SILICONE FLUID SS 4230" et "SS 4267" par la Société GENERAL ELECTRIC et qui sont des "diméthyl/triméthylpolysiloxanes".

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
**a**) des groupements polyéthylèneoxy et/ou polypropylèneoxy, comportant éventuellement des groupes alkyle, tels que :
   - le produit dénommé diméthicone copolyol vendu par la Société DOW CORNING sous les dénominations DC 1248, et l'alkyl (C12) méthicone copolyol vendue par la Société DOW CORNING sous la dénomination Q2 5200,
   - les huiles SILWET L 722, L 7500, L 77, L 711 de la Société UNION CARBIDE,
   - le mélange de diméthicone copolyol et de cyclométhicone tels que le produit vendu sous la dénomination Q2-3225C par la Société DOW CORNING;
**b**) des groupements (per)fluorés tels que des trifluoroalkyles telles que, par exemple, celles vendus par la Société GENERAL ELECTRIC sous les dénominations "FF.150" Fluorosilicone Fluid" ou par la Société SHIN ETSU sous les dénominations X-22-819; X-22-820; X-22-821; X-22-822;
**c**) des groupements hydroxyacylamino telles que celles décrites dans la demande de brevet européen EP-A-0 342 834 et en particulier la silicone vendue par la Société DOW CORNING sous la dénomination "Q2-8413";
**d**) des groupements thiols comme dans les silicones X 2-8360 de la DOW CORNING ou les GP 72A et GP 71 de GENESEE;
**e**) des groupements aminés substitués ou non, comme dans la GP4 SILICONE FLUID de GENESEE, la GP 7100 de GENESEE, la Q2 8220 de DOW CORNING, L'AFL 40 d'UNION CARBIDE ou la silicone dénommée "AMODIMETHICONE" dans le dictionnaire CTFA;
**f**) des groupements carboxylates, comme les produits décrits dans le brevet européen EP 186 507 de CHISSO CORPORATION;
**g**) des groupements hydroxylés, comme les polyorganosiloxanes à fonction hydroxyalkyle, décrits dans la demande de brevet en France n° FR-85 16334, et en particulier les polyorganosiloxanes à fonction γ-hydroxypropyle;
**h**) des groupements alcoxylés comme dans la SILICONE COPOLYMER F 755 de SWS SILICONES et les produits ABILWAX 2428, ABILWAX 2434, ABILWAX 2440 de la Société GOLDSCHMIDT;
**i**) des groupements acyloxyalkyle, comme par exemple les polyorganopolysiloxanes décrits dans la demande de brevet français n° 88 17433, et en particulier les polyorganosiloxanes à fonction stéaroyloxypropyle.
**j**) des groupements ammonium quaternaire, comme dans les produits X2 81 08 et X2 81 09, le produit ABIL K 3270 de la Société GOLDSCHMIDT;
**k**) des groupements amphotères ou bétaïniques, tels que dans le produit vendu par la Société GOLDSCHMIDT sous la dénomination ABIL B 9950.
**l**) des groupements bisulfite, tels que dans les produits vendus par la Société GOLDSCHMIDT sous les dénominations ABIL S 201 et ABIL S 255.

Les alkylgalactoside uronates de formule (I) sont utilisés dans les compositions conformes à l'invention, dans des proportions comprises de préférence entre 1 et 50% en poids par rapport au poids total de la composition.

Les silicones sont utilisées dans les compositions de l'invention dans des proportions comprises de préférence entre 0,01 et 20% en poids par rapport au poids total de la composition.

Les compositions conformes à l'invention peuvent contenir en plus un agent épaississant et/ou un agent de mise en suspension de la silicone tels que des alcalnolamides d'acides gras, des acides polyacryliques, des dérivés de cellulose, des esters d'acides gras et de polyéthylèneglycol, les copolymères réticulés d'acrylamide et d'un monomère choisi parmi l'acrylate d'ammonium, l'acide 2-acrylamido 2-méthylpropane sulfonique partiellement ou totalement neutralisé ou le chlorure de méthacryloyloxyéthyl triméthylammonium; les uréthannes polyéthers, les copolymères de méthylvinyléther/acide maléïque réticulés .

On peut également citer comme agent de mise en suspension de la silicone les composés choisis parmi
a) ceux de formule :

   R₃X (II)

   dans laquelle R₃ est un radical aliphatique à longue chaîne carbonée, éventuellement interrompue par un ou plusieurs atomes d'oxygène, et X est un reste acide carboxylique, sulfurique ou phosphorique ou un radical dérivé d'un acide carboxylique ou d'un amide; ces composés de formule (II) sont choisis parmi ceux dans lesquels :
   (i) R₃ est un radical alkyle ou alcényle en C₁₁-C₂₁;
      et X est
      - un groupement COOA où A est un radical mono- ou polyhydroxyalkyle dérivé d'un polyol en C₂-C₃ ou un radical CH₂CH₂SO₃M;
      - un groupement CO(OCH₂CH₂)ₖOH où k a une valeur comprise entre 2 et 150;
      - un groupement 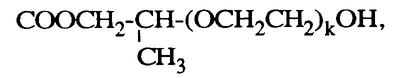
      - où k a une valeur comprise entre 2 et 150, les fonctions OH libres des groupements définis ci-dessus pouvant être estérifiées par un acide RCOOH où R' est un alkyle ou alcényle en C₁₁-C₂₁;
      - un groupement CONR₄R₅ où R₄ et R₅ représentent hydrogène ou hydroxyalkyle en C₁-C₄, l'un au moins représentant hydroxyalkyle en C₁-C₄;
      - un groupement OSO₃M ou 1/3 PO₄ ³⁻ M₃ où M représente un métal alcalin, ammonium ou un reste d'alcanolamine en C₁-C₄;
   (ii) R₃ désigne un radical R₆O(C₂H₄O)ₗ CH₂ et X désigne un groupement COOM où M a la signification indiquée ci-dessus, R₆ désignant un radical alkyle en C₁₂-C₁₄ et l un nombre entier ou décimal compris entre 2,5 et 10, ou bien R₃ désigne oléyle et l varie de 2 à 9 ou encore R₆ désigne alkyle compris en (C₈-C₉) phényle et l varie de 4 à 8 , ou les dérivés dans lesquels R₆ désigne un groupement alkyle(C₁₂-C₁₆) et X un groupement CONR₄R₅; dans lequel R₄ et R₅ ont la même signification que celle indiquée ci-dessus et l a une valeur de 1 à 3 inclus;
b) des oxydes d'amines de formule : 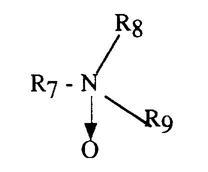 dans laquelle R₇ désigne un groupement alkyle en C₁₆-C₂₂, et R₈ et R₉, identiques ou différents, représentent un groupement alkyle en C₁-C₄ ou hydroxyalkyle en C₁-C₄;
c) les biopolysaccharides choisis parmi les gommes de xanthane et les scléroglucanes;

Si les compositions selon l'invention ne sont pas utilisées pour le lavage des matières kératiniques, la concentration en tensio-actifs anioniques de formule (I) est comprise entre 1 et 10% et plus particulièrement entre 1 et 5% en poids par rapport au poids total de la composition. Ces compositions sont utilisées notamment comme compositions à rincer ou non, appliquées avant ou après shampooing, une coloration, une décoloration, une permanente, un défrisage ou dans des compositions de décoloration, de coloration, de permanente ou de défrisage.

Lorsque les compositions selon l'invention sont des compositions lavantes, elles contiennent les agents tensio-actifs de formule (I) dans une concentration comprise entre 4 et 50% en poids et de préférence entre 8 et 40% en poids par rapport au poids total de la composition.

Les compositions peuvent contenir en plus des agents tensio-actifs supplémentaires de nature anionique, non-ionique, amphotère, zwitterionique ou cationique.

Parmi les agents tensio-actifs anioniques, on peut citer les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkyléthersulfonates, les alkylsulfonates, les alkylamides sulfonates, les alkylarylsulfonates, les oléfines sulfonates, les paraffines sulfonates; les alkylsulfosuccinates, les alkyléthersulfosuccinates, les alkylamides sulfosuccinates; les alkylsulfosuccinamates; les acylglutamates; les alkylsulfoacétates; les alkylétherphosphates; les acylsarcosinates; les N-acyltaurates; les iséthionates; les alkylamides sulfates.

Le radical alkyle ou acycle de ces différents composés est généralement constitué par une chaîne carbonée comportant de 10 à 20 atomes de carbone.

On peut également utiliser des agents tensio-actifs faiblement anioniques, tels que les acides alkylamides ou alkyléthers carboxyliques polyoxyalkylénés, tels que ceux comportant 2 à 50 groupements oxyde d'éthylène.

Les agents tensio-actifs non-ioniques sont plus particulièrement choisis parmi les alcools ou les α-diols ou les alkylphénols ou les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amines grasses polyéthoxylées ayant de préférence 2 à 30 moles d'oxyde d'éthylène; les esters d'acides gras de sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols; les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

On peut également citer les alkylpeptides, les alkylimidazolium bétaïnes.

Parmi les dérivés d'amines, on peut citer les produits commercialisés sous la dénomination MIRANOL, tels que ceux décrits dans les brevets US-A-2 528 378 et 2 781 354 ou classés dans le dictionnnaire CTFA, 3ème édition, 1982, sous les dénominations d'Amphocarboxyglycinates ou d'Amphocarboxypriopionates.

Les agents tensio-actifs cationiques sont choisis parmi les sels d'ammonium quaternaires tels que les halogénures d'alkyl(C₈-C₂₂) triméthyl ammonium, les halogénures de dialkyl(C₈-C₂₂)diméthyl ammonium, les halogénures d'alkyl(C₈-C₂₂)diméthylhydroxyéthylammonium.

Les co-tensio-actifs additionnels peuvent représenter jusqu'à 50% du poids total des tensio-actifs présents dans la composition.

Le pH des compositions conforme à l'invention est généralement compris entre 2 et 10,5 plus particulièrement entre 3 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C₁-C₄ comme l'éthanol, l'isopropanol, le n-butanol; les alkylèneglycols comme le propylèneglycol, les éthers de glycols.

Les compositions selon l'invention peuvent se présenter sous forme de liquides plus ou moins épaissis, de gels, d'émulsions (laits ou crèmes), de lotions hydroalcooliques, de dispersions, de mousses aérosol ou de pains solides.

Les compositions sont par exemple des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, des fards et fonds de teint pour le visage, des vernis à ongles, des shampooings, des produits pour le bain ou la douche, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions conformes à l'invention peuvent également contenir en plus divers additifs tels que des renforçateurs de mousse, des séquestrants, des parfums, des électrolytes, des corps gras tels que des alcools gras, des céramides, des huiles ou cires minérales, végétales, animales ou synthétiques, des filtres UV, des agents anti-radicaux libres, des agents nacrants, des biocides, des antibactériens, des agents antipelliculaires, des agents anti-séborrhéïques, des anti-parasitaires, des repellents, des colorants, des pigments, des oxydants, des réducteurs, des hydratants, des polymères anioniques et non ioniques, des vitamines, des α-hydroxyacides.

Le procédé de lavage et/ou de conditionnement des matières kératiniques et en particulier des cheveux ou de la peau conforme à l'invention, consiste à appliquer sur ces matières au moins une composition telle que définie ci-dessus, cette application étant suivie éventuellement d'une étape de rinçage à l'eau.

Les compositions de lavage peuvent être utilisées comme shampooing mais également comme gel-douche pour le lavage des cheveux et de la peau, auquel cas ils sont, appliqués sur la peau et les cheveux humides qui sont rincés après application.

Lorsque les compositions sont utilisées pour le conditionnement des cheveux, elles sont appliquées sur les cheveux humides, après quoi on peut soit les sécher soit après un temps de pose de 1 à 10 minutes, les rincer à l'eau. On constate que les cheveux humides se démêlent bien.

Les exemples qui suivent servent à illustrer l'invention sans toutefois présenter un caractère limitatif.

### EXEMPLE 1

| **SHAMPOOING** | |
|---|---|
| - Décyl - D-galactoside uronate de sodium | 15 g MA |
| - Lauryl éther sulfate de sodium C₁₂/C₁₄ 70/30 2.2OE en solution aqueuse à 28 % vendu sous le nom d'Empicol ESB / 3 FL par Marchon | 5 g MA |
| - Polydiméthyl siloxane de PM 250 000 de viscosité 0,5 m²/s | 3 g |
| - Gomme xanthane vendue sous le nom de Keltrol T par Kelco | 1 g |
| Colorants, parfum, conservateur | |
| eau qsp | 100 g |
| pH ajusté à 7 par NaOH | |
| L'aspect de ce shampooing est opalescent et visqueux. | |

### EXEMPLE 2

| **GEL DOUCHE** | |
|---|---|
| - Décyl - D-galactoside uronate de sodium | 20 g MA |
| - N-cocoamido ethyl N-éthoxycarboxyméthyl glycinate de sodium | 4 g MA |
| - Mélange de polydiméthyl siloxane α Ω dihydroxylé/cyclotetra et cyclo penta diméthyl siloxane (56/44) (13/87) vendu par DOW CORNING sous le nom de Q2 1401 | 2 g |
| - Gomme de xanthane vendue sous le nom de Keltrol T par Kelco | 0,75 g |
| Colorants, parfum, conservateur | |
| eau qsp | 100 g |
| pH ajusté à 7 par HCl | |
| L'aspect de ce gel est opalescent et visqueux. | |

### EXEMPLE 3

| **BAIN MOUSSANT** | |
|---|---|
| - Décyl - D-galactoside uronate de sodium | 25 g MA |
| - Lauryl éther sulfate de sodium C₁₂/C₁₄ 70/30 2.2OE en solution aqueuse à 28 % vendu sous le nom d'Empicol ESB / 3 FL par Marchon | 5 g MA |
| - Mélange de polydiméthyl siloxane PM 500 000 / et de polydiméthylsiloxane - PM 1000 (33/67) vendu sous la dénomination CF 1241 par la Société Général Electric | 1 g |
| - Suif oxyéthyléné (60 OE) éther de myristyl glycol vendu sous le nom d'elfacos GT 282 S par la société AKZO | 2 g MA |
| Colorants, parfum, conservateur | |
| eau qsp | 100 g |
| pH ajustéé à 8 par NaOH | |
| On obtient un bain moussant opalescent et légèrement visqueux. | |

## Revendications

1. Composition cosmétique, caractérisé par le fait qu'elle contient dans un milieu aqueux cosmétiquement acceptable :
(**A**) au moins un agent tensio-actif anionique du type alkylgalactoside uronate de formule : 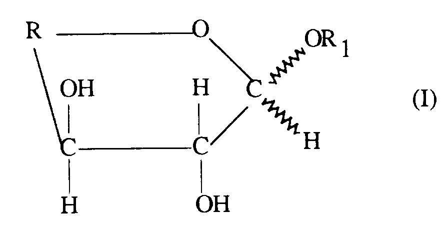 dans laquelle :
R₁ désigne un radical alkyle linéaire ou ramifié en C₈-C₂₂
R désigne un groupe
(i) 〉CH -CH(OH) - CO₂R₂ ou
(ii) 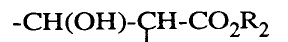 dont le carbone portant le groupe hydroxyle est relié à l'atome d'oxygène endocyclique; R₂ désigne un hydrogène, un métal alcalin, un métal alcalino-terreux ou un groupement ammonium quaternaire non substitué ou substitué par des radicaux alkyle, hydroxyalkyle ou dérivé d'amino-acide.
(**B**) au moins un organopolysiloxane choisi parmi :
(i) les huiles d'orgonapolysiloxanes à l'exclusion des polydiméthyl siloxanes linéaires de viscosité inférieure à 10⁻¹m²/s,
(ii) les gommes d'organopolysiloxanes,
(iii) les résines d'organopolysiloxanes,
(iv) les polysiloxanes organomodifiés,
(v) les mélanges de polydiméthylsiloxanes linéaires de viscosité inférieure à 10⁻¹m²/s avec un ou plusieurs composé(s) défini(s) sous (i), (ii), (iii) et (iv).

2. Composition selon la revendication 1, caractérisée par le fait que dans la formule (I), le radical R₂ désigne le sodium ou le potassium; le magnésium; le groupe ammonium quaternaire dérivé d'ammoniaque, de triéthanolamine, de monoéthanolamine, de 2-amino 2-méthyl 1,3-propanediol, de 2-méthyl 2-amino 1-propanol, d'histidine, d'arginine, ou de lysine.

3. Composition selon la revendication 1 ou 2, caractérisé par le fait que le composé de formule (I) est choisi parmi ceux pour lesquels R₁ désigne un alkyle en C₈-C₁₄.

4. Composition selon l'une quelconque des revendications 1 à 3, caractérisée par le fait que les composés de formule (I) sont choisis parmi ceux pour lesquels R₁ désigne le radical décyle.

5. Composition selon la revendication 4, caractérisée par le fait que le composé de formule (I) est choisi parmi :
le décyl α-D-galactopyranoside uronate de sodium
le décyl β-D-galactopyranoside uronate de sodium
le décyl α-D-galactofuranoside uronate de sodium
le décyl β-D-galactofuranoside uronate de sodium.

6. Composition selon l'une quelconque des revendications 1 à 5, caractérisée par le fait que l'organosiloxane est une silicone volatile ayant un point d'ébullition compris entre 60°C et 260°C, choisie parmi : les silicones cycliques de 3 à 7 atomes de silicium et de préférence 4 à 5 ou les cyclocopolymères du type diméthylsiloxane/méthylalkylsiloxane,

7. Composition selon l'une quelconque des revendications 1 à 5, caractérisée par le fait que l'organopolysiloxane est une silicone non volatile choisie parmi les polyalkylsiloxanes, les polyarylsiloxanes, les polyalkylarylsiloxanes, les gommes et résines de silicone, les polysiloxanes organomodifiés, ainsi que leurs mélanges.

8. Composition selon la revendication 7, caractérisée par le fait que l'organopolysiloxane est choisi parmi :
A) les polyalkyl(C₁-C₂₀)siloxanes; les polydiméthylsiloxanes linéaires à groupements terminaux triméthylsilyle et les polydiméthylsiloxanes linéaires à groupements terminaux hydroxydiméthylsilyle, de viscosité supérieure à 10⁻¹m²/s;
B) les polyméthylphénylsiloxanes, les polydiméthylméthylphénylsiloxanes, polydiméthyldiphénylsiloxanes linéaires et/ou ramifiés;
C) les gommes de masse moléculaire comprise entre 200 000 et 1 000 000, utilisées seules ou sous forme de mélange dans un solvant, choisies dans le groupe constitué par les copolymères suivants :
- polydiméthylsiloxane
- poly[(diméthylsiloxane)/(méthylvinylsiloxane)],
- poly[(diméthylsiloxane)](diphénylsiloxane)],
- poly[(diméthylsiloxane))(phénylméthylsiloxane)],
- poly[(diméthylsiloxane)/(diphénylsiloxane)/(méthylvinylsiloxane)].
D) les résines d'organopolysiloxanes renfermant les unités R₂SiO_{2/2}, RSiO_{3/2} et SiO_{4/2} dans lesquelles R représente un groupement hydrocarboné possédant 1 à 6 atomes de carbone ou un groupement phényle.
E) les organopolysiloxanes comportant dans leur structure générale un ou plusieurs groupement(s) organofonctionnel(s) directement fixé(s) sur la chaîne siloxanique ou fixé(s) par l'intermédiaire d'un radical hydrocarboné, choisis parmi les polyorganosiloxanes comportant :
a) des groupemenst polyéthylèneoxy et/ou propylèneoxy comportant éventuellement des groupes alkyle;
b) des groupes (per)fluorés;
c) des groupes hydroxyacylamino;
d) des groupements thiols;
e) des groupes aminés substitués ou non;
f) des groupements carboxylates;
g) des groupements hydroxylés;
h) des groupements alcoxylés;
i) des groupements acyloxyalkyle;
j) des groupements ammonium quaternaire;
k) des groupements amphotères ou bétaïniques
l) des groupements bisulfite.

9. Composition selon l'une quelconque des revendications 1 à 8, caractérisée par le fait que les agents tensio-actifs de formule (I) sont présents dans des proportions comprises entre 1 à 50% en poids et que les silicones sont présentes dans des proportions comprises entre 0,01 et 20% en poids; les pourcentages en poids étant exprimés par rapport au poids total de la composition.

10. Composition de conditionnement des matières kératiniques selon l'une quelconque des revendications 1 à 9, caractérisé par le fait que la concentration en agents tensio-actifs anioniques de formule (I) est comprise entre 1 et 10% en poids par rapport au poids total de la composition.

11. Composition de lavage des matières kératiniques selon l'une quelconque des revendications 1 à 9, caractérisée par le fait que la concentration en tensio-actifs anioniques de formule (I) est comprise entre 4 et 50% en poids par rapport au poids total de la composition.

12. Composition selon les revendications 1 à 11, caractérisée par le fait qu'elle contient en plus un co-tensio-actif additionnel du type anionique, non-ionique, amphotère ou cationique dans une proportion allant jusqu'à 50% du poids total en agents tensio-actifs.

13. Composition selon la revendication 12, caractérisée par le fait que le co-tensio-actif anionique additionnel est choisi parmi les sels alcalins, les sels d'ammonium, les sels d'amines ou d'aminoalcools ou les sels de magnésium des composés : acides gras; alkylsulfates, alkyléthersulfates, alkylamidoéthersulfates, alkylarylpolyéthersulfates, monoglycérides sulfates, alkylamides sulfates; alkylsulfonates, alkyléthersulfonates, alkylsulfosuccinates, alkyléthersulfosuccinates, alkylamides sulfosuccinates; acylglutamates; alkylsulfosuccinamates; alkylsulfoacétates; alkylétherphosphates; acylsarcosinates; N-acyltaurates; isethionates; le radical alkyle ou acyle étant constitué d'une chaîne carbonée compôrtant de 10 à 20 atomes de carbone ou bien des acides alkylamides ou alkyléthers carboxyliques polyoxyalkylénés.

14. Composition selon la revendication 12, caractérisée par le fait que le co-tensio-actrif non-ionique est choisi parmi les alcools, les α-diols, les alkylphénols et les acides gras polyéthoxylés ou polypropoxylés à chaine grasse comportant 8 à 18 atomes de carbone, le nombre de groupes oxyde d'éthylène ou oxyde de propylène étant compris entre 2 et 50 et le nombre de groupes glycérol étant compris entre 2 et 30; les copolymères d'oxydes d'éthylène et de propylène; les condensats d'oxyde d'éthylène et de propylène sur des alcools gras; les amides gras polyéthoxylés; les amines grasses polyéthoxylées; les esters d'acides gras de sucre; les esters d'acides gras du polyéthylèneglycol; les esters d'acides gras de glycols; les oxydes d'amines.

15. Composition selon la revendication 12, caractérisée par le fait que le co-tensio-actif amphotère ou zwitterionique additionnel est choisi parmi les dérivés d'amines secondaires ou tertiaires aliphatiques dans lesquels le radical aliphatique est une chaine linéaire ou ramifiée comportant 8 à 18 atomes de carbone et qui contient au moins un groupe anionique hydrosolubilisant carboxylate, sulfonate, sulfate, phosphate ou phosphonate; les alkyl (C₈-C₂₀) bétaïnes; les sulfobétaïnes, les alkyl (C₈-C₂₀)amidoalkyl (C₁-C₆)bétaïnes ou les alkyl(C₈-C₂₀)amidoalkyl(C₁-C₆) sulfobétaïnes; les alkylpeptides; les alkylimidazoliumbétaïnes.

16. Composition de lavage selon la revendication 12, caractérisée par le fait que le co-tensio-actif cationique additionnel est choisi parmi les sels d'ammonium quaternaire.

17. Composition selon l'une quelconque des revendications 1 à 16, caractérisée par le fait qu'elle contient en plus un agent épaisissant et/ou un agent de suspension de la silicone.

18. Composition selon la revendication 17, caractérisée par le fait que l'agent épaississant et/ou l'agent de suspension est choisi parmi les alcanolamides d'acides gras; les acides polyacryliques; les dérivés de cellulose; les esters d'acides gras et de polyéthylèneglycol; les copolymères réticulés d'acrylamide et d'un monomère choisi parmi l'acrylate d'ammonium, l'acide 2-acrylamido 2-méthylpropane sulfonique ou le chlorure de méthacryloyloxyéthyl triméthylammonium; les uréthanes polyéthers; les copolymères de méthylvinyléther/acide maléïque réticulés; les composés suivants :
a) ceux de formule :
R₃X (II)
où R₃ est un radical aliphatique à longue chaîne carbonée, éventuellement interrompue par un ou plusieurs atomes d'oxygène et X est un reste d'acide carboxylique, sulfurique ou phosphorique ou un radical dérivé d'un acide carboxylique ou d'un amide;
b) des oxydes d'amines de formule 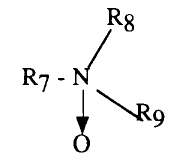 dans laquelle R₇ désigne un alkyle en C₁₆-C₂₂ et R₈ et R₉, identiques ou différents représentent un alkyle en C₁-C₄ ou hydroxyalkyle en C₁-C₄;
c) les biopolysaccharides.

19. Composition selon l'une quelconque des revendications 1 à 18, caractérisée par le fait que le milieu cosmétiquement acceptable est constitué par de l'eau ou un mélange d'eau et d'un solvant cosmétiquement acceptable.

20. Composition selon l'une quelconque des revendications 1 à 19, caractérisée par le fait qu'elle se présente sous forme de liquide plus ou moins épaissi, de gel, d'émulsion, de lotion hydroalcoolique, de dispersion, de pain solide ou de mousse aérosol.

21. Composition selon l'une quelconque des revendications 1 à 20, caractérisée par le fait qu'elle contient en plus des additifs choisis parmi les renforçateurs de mousse, les sequestrants, les électrolytes, parfums, les conservateurs, les alcools gras, les huiles ou cires minérales, végétales, animales ou synthétiques, les céramides, les filtres UV, les agents nacrants, les anti-radicaux libres, les biocides, les antibactériens, les agents antipelliculaires, antiséborrhéïques, antiparasitaires, les repellents, des colorants, des pigments, des oxydants, des réducteurs, des hydratants, des polymères anioniques ou non-ioniques, des vitamines ou des α-hydroxyacides.

22. Utilisation cosmétique de la composition telle que définie dans l'une quelconque des revendications 1 à 21 pour le traitement et/ou le lavage des matières kératiniques constituées par les cheveux ou la peau.

23. Procédé de lavage et/ou de conditionnement cosmétique des cheveux ou de la peau, caractérisé par le fait qu'il consiste à appliquer sur la peau ou les cheveux une quantité efficace de composition selon l'une quelconque des revendications 1 à 21, cette application étant éventuellement suivie d'un rinçage à l'eau.

## Claims

1. Cosmetic composition, characterized in that it contains, in a cosmetically acceptable aqueous medium:
(**A**) at least one anionic surfactant of alkylgalactoside uronate type of formula: 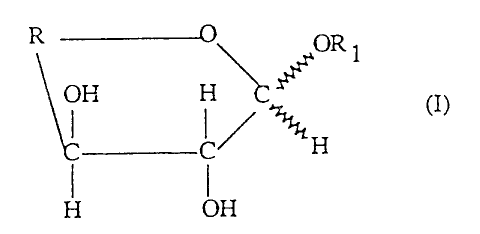 in which:
R₁ denotes a linear or branched C₈-C₂₂ alkyl radical,
R denotes a group
(i) 〉CH -CH(OH) - CO₂R₂ or
(ii) 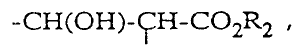 in which the carbon carrying the hydroxyl group is connected to the endocyclic oxygen atom; R₂ denotes a hydrogen, an alkali metal, an alkaline-earth metal or a quaternary ammonium group which is unsubstituted or substituted by alkyl or hydroxyalkyl radicals or derived from an amino acid,
(**B**) at least one organopolysiloxane chosen from:
(i) organapolysiloxane oils with the exception of linear polydimethylsiloxanes with a viscosity of less than 10⁻¹ m²/s,
(ii) organopolysiloxane gums,
(iii) organopolysiloxane resins,
(iv) organomodified polysiloyanes,
(v) mixtures of linear polydimethylsiloxanes with a viscosity of less than 10⁻¹ m²/s with one or a number of compound(s) defined under (i), (ii), (iii) and (iv).

2. Composition according to Claim 1, characterized in that, in the formula (I), the radical R₂ denotes sodium or potassium; magnesium; or the quaternary ammonium group derived from ammonia, triethanolamine, monoethanolamine, 2-amino-2-methyl-1,3-propanediol, 2-methyl-2-amino-1-propanol, histidine, arginine or lysine.

3. Composition according to Claim 1 or 2, characterized in that the compound of formula (I) is chosen from those in which R₁ denotes a C₈-C₁₄ alkyl.

4. Composition according to any one of Claims 1 to 3, characterized in that the compounds of formula (I) are chosen from those in which R₁ denotes the decyl radical.

5. Composition according to Claim 4, characterized in that the compound of the formula (I) is chosen from:
sodium decyl α-D-galactopyranoside uronate
sodium decyl β-D-galactopyranoside uronate
sodium decyl α-D-galactofuranoside uronate
sodium decyl β-D-galactofuranoside uronate.

6. Composition according to any one of Claims 1 to 5, characterized in that the organosiloxane is a volatile silicone having a boiling point of between 60°C and 260°C chosen from: cyclic silicones containing 3 to 7 silicon atoms and preferably 4 to 5 or cyclocopolymers of the dimethylsiloxane/methylalkylsiloxane type.

7. Composition according to any one of Claims 1 to 5, characterized in that the organopolysiloxane is a nonvolatile silicone chosen from polyalkylsiloxanes, polyarylsiloxanes, polyalkylarylsiloxanes, silicone gums and resins and organomodified polysiloxanes, as well their mixtures.

8. Composition according to Claim 7, characterized in that the organopolysiloxane is chosen from:
A) poly(C₁-C₂₀)alkylsiloxanes; linear polydimethylsiloxanes containing trimethylsilyl end groups and linear polydimethylsiloxanes containing hydroxydimethylsilyl end groups, with a viscosity greater than 10⁻¹ m²/s;
B) linear and/or branched polymethylphenylsiloxanes, polydimethylmethylphenylsiloxanes or polydimethyldiphenylsiloxanes;
C) gum with a molecular mass of between 200,000 and 1,000,000, used alone or in the form of a mixture in a solvent, chosen from the group consisting of the following copolymers:
- polydimethylsiloxane,
- poly[(dimethylsiloxane)/(methylvinylsiloxane)],
- poly[(dimethylsiloxane)](diphenylsiloxane)] [sic],
- poly[(dimethylsiloxane))(phenylmethylsiloxane)] [sic],
- poly[(dimethylsiloxane)/(diphenylsiloxane)/(methylvinylsiloxane)].
D) organopolysiloxane resins containing units R₂SiO_{2/2}, RSiO_{3/2} and SiO_{4/2} in which R represents a hydrocarbon group having 1 to 6 carbon atoms or a phenyl group;
E) organopolysiloxanes containing in their general structure one or a number of organofunctional group(s) attached directly to the siloxane chain or attached via a hydrocarbon radical chosen from polyorganosiloxanes containing:
a) polyethyleneoxy and/or propyleneoxy groups optionally containing alkyl groups;
b) (per)fluorinated groups;
c) hydroxyacylamino groups;
d) thiol groups;
e) substituted or unsubstituted amino groups;
f) carboxylate groups;
g) hydroxylated groups;
h) alkoxylated groups;
i) acyloxyalkyl groups;
j) quaternary ammonium groups;
k) amphoteric or betaine groups;
l) bisulfite groups.

9. Composition according to any one of Claims 1 to 8, characterized in that the surfactants of formula (I) are present in proportions of between 1 to 50% by weight and in that the silicones are present in proportions of between 0.01 and 20% by weight; the percentages by weight being expressed with respect to the total weight of the composition.

10. Composition for conditioning keratinous substances according to any one of Claims 1 to 9, characterized in that the concentration of anionic surfactants of formula (I) is between 1 and 10% by weight with respect to the total weight of the composition.

11. Composition for washing keratinous substances according to any one of Claims 1 to 9, characterized in that the concentration of anionic surfactants of formula (I) is between 4 and 50% by weight with respect to the total weight of the composition.

12. Composition according to Claims 1 to 11, characterized in that it furthermore contains an additional cosurfactant of anionic, nonionic, amphoteric or cationic type in a proportion ranging up to 50% of the total weight of surfactants.

13. Composition according to Claim 12, characterized in that the additional anionic cosurfactant is chosen from the alkali metal salts, the ammonium salts, the amine or aminoalcohol salts or the magnesium salts of the compounds: fatty acids; alkyl sulfates, alkyl ether sulfates, alkylamidoether sulfates, alkylarylpolyether sulfates, monoglyceride sulfates or alkylamide sulfates; alkylsulfonates, alkylethersulfonates, alkylsulfosuccinates, alkylethersulfosuccinates or alkylamidesulfosuccinates; acylglutamates; alkylsulfosuccinamates; alkylsulfoacetates; alkyl ether phosphates; acylsarcosinates; N-acyltaurates; or isethionates; the alkyl or acyl radical consisting of a carbon chain containing from 10 to 20 carbon atoms or else polyoxyalkylenated alkyl amide or alkyl ether carboxylic acids.

14. Composition according to Claim 12, characterized in that the nonionic cosurfactant is chosen from the polyethoxylated or polypropoxylated alcohols, α-diols, alkylphenols and fatty acids, with a fatty chain containing 8 to 18 carbon atoms, the number of ethylene oxide or propylene oxide groups being between 2 and 50 and the number of glycerol groups being between 2 and 30 [sic]; the copolymers of ethylene oxide and of propylene oxide; the condensates of ethylene oxide and of propylene oxide with fatty alcohols; the polyethoxylated fatty amides; the polyethoxylated fatty amines; the fatty acid esters of sugar ; the fatty acid esters of polyethylene glycol; the fatty acid esters of glycols; or the amine oxides.

15. Composition according to Claim 12, characterized in that the additional amphoteric or zwitterionic cosurfactant is chosen from the derivatives of secondary or tertiary aliphatic amines, in which the aliphatic radical is a linear or branched chain containing 8 to 18 carbon atoms and which contains at least one water-solubilizing carboxylate, sulfonate, sulfate, phosphate or phosphonate anionic group; the (C₈-C₂₀)alkylbetaines, the sulfobetaines, the (C₈-C₂₀)alkylamido(C₁-C₆)alkylbetaines or the (C₈-C₂₀)alkylamido(C₁-C₆)alkylsulfobetaines; the alkylpeptides; or the alkylimidazoliumbetaines [sic].

16. Washing composition according to Claim 12, characterized in that the additional cationic cosurfactant is chosen from quaternary ammonium salts.

17. Composition according to any one of Claims 1 to 16, characterized in that it additionally contains a thickening and/or a suspending agent for the silicone.

18. Composition according to Claim 17, characterized in that the thickening agent and/or the suspending agent is chosen from fatty acid alkanolamides; poly(acrylic acid)s; cellulose derivatives; esters of fatty acids and of polyethylene glycol; crosslinked copolymers of acrylamide and of a monomer chosen from ammonium acrylate, 2-acrylamido-2-methylpropanesulfonic acid or methacryloyloxyethyltrimethylammonium chloride; polyurethanes; crosslinked methyl vinyl ether-maleic acid copolymers; or the following compounds:
a) those of formula:
R₃X (II)
where R₃ is an aliphatic radical with a long carbon chain, optionally interrupted by one or a number of oxygen atoms, and X is a carboxylic, sulfuric or phosphoric acid residue or a radical derived from a carboxylic acid or from an amide;
b) amine oxides of formula 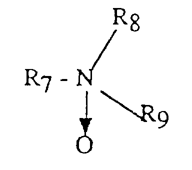 in which R₇ denotes a C₁₆-C₂₂ alkyl and R₈ and R₉, which are identical or different, represent a C₁-C₄ alkyl or C₁-C₄ hydroxyalkyl;
c) biopolysaccharides.

19. Composition according to any one of Claims 1 to 18, characterized in that the cosmetically acceptable medium consists of water or a mixture of water and of a cosmetically acceptable solvent.

20. Composition according to any one of Claims 1 to 19, characterized in that it is provided in the form of a more or less thickened liquid, a gel, an emulsion, an aqueous/alcoholic lotion, a dispersion, a solid bar or an aerosol foam.

21. Composition according to any one of Claims 1 to 20, characterized in that it furthermore contains additives chosen from foam reinforcers, sequestering agents, electrolytes, fragrances, preservatives, fatty alcohols, mineral, vegetable, animal or synthetic oils or waxes, ceramides, UV screening agents, pearlescence agents, agents for combating free radicals, biocides, antibacterials, antidandruff, antiseborrheic or antiparasitic agents, repellents, dyes, pigments, oxidizing agents, reducing agents, moisturizers, anionic or nonionic polymers, vitamins or α-hydroxy acids.

22. Cosmetic use of the composition as defined in any one of Claims 1 to 21 for treating and/or washing keratinous susbtances consisting of the hair or the skin.

23. Process for cosmetic washing and/or conditioning of the hair or of the skin, characterized in that it consists in applying an effective amount of composition according to any one of claims 1 to 21 to the skin or the hair, this application optionally being followed by rinsing with water.

## Patentansprüche

1. Kosmetische Zusammensetzung,
dadurch **gekennzeichnet**, daß
sie in einem kosmetisch geeigneten wässrigen Milieu enthält:
(A) mindestens ein anionisches oberflächenaktives Mittel vom Alkylgalactosiduronat-Typ der Formel: 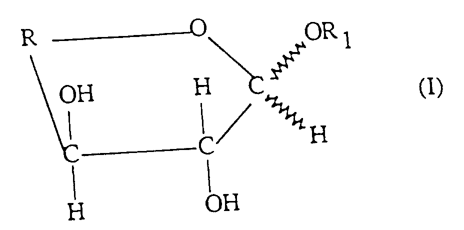 worin gilt:
R₁ bedeutet einen linearen oder verzweigten Alkylrest mit 8 bis 22 Kohlenstoffatomen,
R bedeutet eine Gruppe:
(i) 〉CH -CH(OH) - CO₂R₂ oder
(ii) 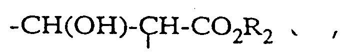 deren die Carboxylgruppe aufweisender Kohlenstoff an das endozyklische Sauerstoffatom gebunden ist, und worin R₂ Wasserstoff, ein Alkalimetall, Erdalkalimetall oder eine gegebenenfalls mit Alkyl-, Hydroxyalkyl- oder von Aminosäuren abgeleiteten Resten substituierte Ammoniumgruppe ist,
(B) mindestens ein Organopolysiloxan, ausgewählt aus:
(i) - Organopolysiloxanölen, ausgenommen lineare Polydimethylsiloxane einer Viskosität von weniger als 10⁻¹ m²/S,
(ii) Gummiprodukten aus Organopolysiloxanen,
(iii) Organopolysiloxanharzen,
(iv) organomodifizierten Polysiloxanen und aus
(v) Mischungen aus linearen Polydimethylsiloxanen einer Viskosität von Weniger als 10⁻¹ m²/s mit einer oder mehreren unter (i), (ii), (iii) und (iv) definierten Verbindungen.

2. Zusammensetzung gemäß Anspruch 1,
dadurch **gekennzeichnet**, daß
in der Formel (I) der Rest R₂ Natrium oder Kalium, Magnesium oder eine quaternäre Ammoniumgruppe bedeutet, die von Ammoniak, Triethanolamin, Monoethanolamin, 2-Amino-2-methylpropan-1,3-diol, 2-Methyl-2-aminopropan-1-ol, Histidin, Arginin oder Lysin abgeleitet ist.

3. Zusammensetzung gemäß Anspruch 1 oder 2,
dadurch **gekennzeichnet**, daß
die Verbindung der Formel (I) aus denjenigen ausgewählt ist, für die R₁ einen C₈₋₁₄-Alkylrest bedeutet.

4. Zusammensetzung gemäß einem der Ansprüche 1-3,
dadurch **gekennzeichnet**, daß
die Verbindungen der Formel (I) aus denjenigen ausgewählt ist, für die R₁ den Decylrest bedeutet.

5. Zusammensetzung gemäß Anspruch 4,
dadurch **gekennzeichnet**, daß
die Verbindung der Formel (I) ausgewählt ist aus:
Natrium-Decyl-α-D-galactopyranosiduronat,
Natrium-Decyl-β-D-gaiaotopyranosiduronat,
Natrium-Decyl-α-D-galactofuranosiduronat und aus
Natrium-Decyl-β-D-galactofuranosiduronat.

6. Zusammensetzung gemäß einem der Ansprüche 1 bis 5,
dadurch **gekennzeichnet**, daß
das Organosiloxan ein fiüchtiges Silicon mit einem Siedepunkt von 60 bis 260°C ist, ausgewählt aus: zyklischen Siliconen mit 3 bis 7 und vorzugsweise mit 4 bis 5 Siliziumatomen oder aus Cyclocopolymeren vom Dimethylsiloxan/Methylalkylsiloxan-Typ.

7. Zusammensetzung gemäß einem der Ansprüche 1 bis 5,
dadurch **gekennzeichnet**, daß
das Organopolysiloxan ein nicht-flüchtiges Silicon ist, das aus Polyalkylsiloxanen, Polyarylsiloxanen, Polyalkylarylsiloxanen, Gummi- und Harzprodukten aus Silicon, organomodifizierten Polysiloxanen sowie aus deren Mischungen ausgewählt ist.

8. Zusammensetzung gemäß Anspruch 7,
dadurch **gekennzeichnet**, daß
das Organopolysiloxan ausgewählt ist aus:
A) C₁₋₂₀-Polyalkylsiloxanen, linearen Polydimethylsiloxanen mit endständigen Trimethylsilylgruppen und aus linearen Polydimethylsiloxanen mit endständigen Hydroxydimethylsilylgruppen, welche eine Viskosität von mehr als 10⁻¹ m²/s aufweisen;
B) aus linearen und oder verwzeigten Polymethylphenyl-, Polydimethylmethylphenyl- und aus Polydimethyldiphenylsiloxanen;
C) aus Gummiprodukten einer Molekularmasse von 200 000 bis 1 000 000, welche alleine oder in Form einer Mischung in einem Lösungsmittel verwendet werden, welche aus der Gruppe aus den folgenden Copolymeren ausgewählt sind:
- Polydimethylsiloxan,
- Poly((dimethylsiloxan)/(methylvinylsiloxan)),
- Poly((dimethylsiloxan)/(diphenylsiloxan)),
- Poly((dimethylsiloxan)/(phenylmethylsiloxan)) und aus
- Poly((dimethylsiloxan)/(diphenylsiloxan)/(methylvinyl-Siloxan));
D) aus Organopolysiloxan-Harzen, enthaltend die Einheiten R₂SiO_{2/2}, RSiO_{3/2} und SiO_{4/2}, in denen R eine Kohlenwasserstoffgruppe mit 1 bis 6 Kohlenstoffatomen oder eine Phenylgruppe darstellt;
E) aus Organopolysiloxanen, die in ihrer allgemeinen Struktur eine oder mehrere organofunktionelle Gruppierungen aufweisen, die direkt oder über das Zwischenglied eines Kohlenwasserstoffrestes an die Siloxankette gebunden sind, welche aus Polyorganosiloxanen ausgewählt sind, die aufweisen:
a) Polyethylen- und/oder Polypropylenoxygruppen mit gegebenenfalls Alkylgruppen;
b) (per)fluorierte Gruppen,
c) Hydroxyacylaminogruppen;
d) Thiolgruppen;
e) gegebenenfalls substituierte Amingruppen;
f) Carboxylatgruppen;
g) Hydroxylgruppen;
h) Alkoxylgruppen;
i) Acyloxyalkylgruppen;
j) quaternäre Ammoniumgruppen;
k) amphotere oder betainische Gruppen und
l) Bisulfit-Gruppen.

9. Zusammensetzung gemäß einem der Ansprüche 1 bis 8,
dadurch **gekennzeichnet**, daß
die oberflächenaktiven Mittel der Formel (I) in Mengenanteilen von 1 bis 50 Gew.% und die Silicone in Mengenanteilen von 0,01 bis 20 Gew.% vorhanden sind, wobei die Gew.-Prozentangaben auf das Gesamtgewicht der Zusammensetzung bezogen sind.

10. Zusammensetzung gemäß einem der Ansprüche 1 bis 9 zum Konditionieren keratinischer Materien,
dadurch **gekennzeichnet**, daß
die Konzentration an anionischen oberflächenaktiven Mitteln der Formel (I) 1 bis 10 Gew.% beträgt, bezogen auf das Gesamtgewicht der Zusammensetzung.

11. Zusammensetzung gemäß einem der Ansprüche 1 bis 9 zum Waschen keratinischer Materien,
dadurch **gekennzeichnet**, daß
die Konzentration an anionischen oberflächenaktiven Mitteln der Formel (I) 4 bis 50 Gew.% beträgt, bezogen auf das Gesamtgewicht der Zusammensetzung.

12. Zusammensetzung gemäß einem der Ansprüche 1 bis 11,
dadurch **gekennzeichnet**, daß
sie zusätzlich ein weiteres oberflächenaktives Co-Mittel vom anionischen, nicht-ionischen, amphoteren oder kationischen Typ in einem Mengenanteil von bis zu 50% des Gesamtgewichts an oberflächenaktiven Mitteln enthält.

13. Zusammensetzung gemäß Anspruch 12,
dadurch **gekennzeichnet**, daß
das anionische zusätzliche oberflächenaktive Co-Mittel ausgewählt ist aus Alkali-, Ammonium-, Amin-, Aminoalkohol- oder Magnesiumsalzen der folgenden Verbindungen: von Fettsäuren, Alkylsulfaten, Alkylethersulfaten, Alkylamidoethersulfaten, Alkylarylpolyethersulfaten, Monoglyceridsulfaten, Alkylamidsulfaten, Alkylsulfonaten, Alkylethersulfonaten, Alkylsulfosuccinaten, Alkylethersulfosuccinaten, Alkylamidsulfosuccinaten, Acylglutamaten, Alkylsulfosuccinamaten, Alkylsulfoacetaten, Alkyletherphosphaten, Acylsarcosinaten, N-Acyltauraten, Isethionaten, wobei der Alkyl- oder Acylrest aus einer Kohlenstoffkette mit 10 bis 20 Kohlenstoffatomen oder auch aus polyoxalkylierten Alkylamid- oder Alkylethercarboxylsäuren zusammengesetzt ist.

14. Zusammensetzung gemäß Anspruch 12,
dadurch **gekennzeichnet**, daß
das nicht-ionische oberflächenaktive Co-Mittel aus polyeth- oder polypropoxylierten, eine Fettkette mit 8 bis 18 Kohelnstoffatomen aufweisendenv Alkoholen, α-Diolen, Alkylphenolen und Fettsäuren, wobei die Anzahl an Ethylen- oder Propylenoxidgruppen 2 bis 50 und die Anzahl an Glycerylgruppen 2 bis 30 betragen, aus Copolymeren aus Ethylen- und Propylenoxiden, Kondensaten von Ethylen- und Propylenoxiden mit Fettalkoholen, polyethoxylierten Fettamiden, polyethoxylierten Fettaminen, Zuckerfettsäureestern, Polyethylenglycolfettsäureestern, Fettsäureestern von Glycolen und aus Aminoxiden ausgewählt ist.

15. Zusammensetzung gemäß Anspruch 12,
dadurch **gekennzeichnet**, daß
das zusätzliche amphotere oder zwitterionische oberflächenaktive Co-Mittel aus sekundären oder tertiären aliphatischen Aminderivaten, in denen der aliphatische Rest eine lineare oder verzweigte Kette mit 8 bis 18 Kohlenstoffatomen ist und mindestens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder C₈₋₂₀-Alkylamido-C₁₋₆-alkylsulfobetainen, Alkylpeptiden und aus Alkylimidazoliumbetainen ausgewählt ist.

16. Zusammensetzung gemäß Anspruch 12,
dadurch **gekennzeichnet**, daß
das zusätzliche kationische oberflächenaktive Co-Mittel aus quaternären Ammoniumsalzen ausgewählt ist.

17. Zusammensetzung gemäß einem der Ansprüche 1 bis 16,
dadurch **gekennzeichnet**, daß
sie zusätzlich ein Verdickungsmittel und/oder ein Suspendiermittel für das Silicon enthält.

18. Zusammensetzung gemäß Anspruch 17,
dadurch **gekennzeichnet**, daß
das Verdickungsmittel und/oder Suspendiermittel aus Alkanolamiden von Fettsäuren, Polyacrylsäuren, Cellulosederivaten, Estern von Fettsäure und Polyethylenglycol, aus vernetzten Copolymeren aus Acrylamid und einem Monomer, ausgewählt aus Ammoniumacrylat, 2-Acrylamido-2-methylpropansulfonsäure oder aus Methacryloyloxyethyltrimethylammoniumchlorid, aus Polyetherurethanen, vernetzten Copolymeren von Methylvinylether/Maleinsäure und aus den folgenden Verbindungen ausgewählt sind:
a) aus denjenigen der Formel:
R₃X (II)
worin R₃ ein aliphatischer Rest mit langer Kohlenstoffkette, die gegebenenfalls durch ein oder mehrere Sauerstoffatome unterbrochen ist, und X ein Carboxyl-, Schwefel- oder Phosphorsäurerest oder ein von einer Carboxylsäure oder einem Amid abgeleiteter Rest sind;
b) aus Aminoxiden der Formel: 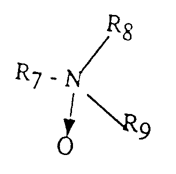 worin R₇ einen C₁₆₋₂₂-Alkylrest und R₈ und R₉, gleich oder verschieden, einen C₁₋₄-Alkyl- oder einen C₁₋₄-Hydroxyalkylrest darstellen;
c) aus Biopolysacchariden.

19. Zusammensetzung gemäß einem der Ansprüche 1 bis 18,
dadurch **gekennzeichnet**, daß
das kosmetisch geeignete Milieu aus Wasser oder aus einer Mischung aus Wasser und einem kosmetisch geeigneten Lösungsmittel zusammengesetzt ist.

20. Zusammensetzung gemäß einem der Ansprüche 1 bis 19,
dadurch **gekennzeichnet**, daß
sie in Form einer mehr oder weniger verdickten Flüssigkeit, eines Gels, einer Emulsion, einer hydroalkoholischen Lotion, einer Dispersion, eines Seifenstücks oder eines Aerosol-Schaums vorliegt.

21. Zusammensetzung gemäß einem der Ansprüche 1 bis 20,
dadurch **gekennzeichnet**, daß
sie zusätzlich Additive enthält, ausgewählt aus Schaumverstärkungsmitteln, Sequestriermitteln, Elektrolyten, Parfüm-Produkten, Konservierungsmitteln, Fettalkoholen, mineralischen, pflanzlichen oder tierischen Ölen, mineralischen, pflanzlichen, tierischen oder synthetischen Wachsen, Ceramiden, UV-Filterstoffen, Perlmuttglanzmitteln, Abfangmitteln für freie Radikale, Biociden, antibakteriellen Mitteln, Mitteln gegen Haarausfall, antiseborrheischen Mitteln, Mitteln gegen Parasiten, Abschreckmitteln, Farbstoffen, Pigmenten, Oxidations-, Reduktionsmitteln, Hydratisiermitteln, anionischen oder nicht-ionischen Polymeren, Vitaminen oder aus α-Hydroxysäuren.

22. Kosmetische Verwendung der in einem der Ansprüche 1 bis 21 definierten Zusammensetzungen zum Behandeln und/oder Waschen keratinischer Materien aus Haaren oder der Haut.

23. Verfahren zum Waschen und/oder kosmetischen Konditionieren der Haare oder der Haut,
dadurch **gekennzeichnet**, daß
man auf die Haut oder die Haare eine ausreichende Menge einer Zusammensetzung gemäß einem der Ansprüche 1 bis 21 aufbringt, worauf auf diese Aufbringung gegebenenfalls eine Spülung mit Wasser erfolgt.
